# FASCICULE DE BREVET EUROPEEN

(11) **EP 2 873 368 B1**
(45) Date de publication et mention de la délivrance du brevet: **14.10.2020**
(21) Numéro de dépôt: 14193370.5
(22) Date de dépôt: 17.11.2014
(51) Int. Cl.: A61B 5/11, A61B 5/08

(54) **Dispositif de détection pour literie pour surveillance du sommeil**
Sensorvorrichtung für Betten zur Überwachung des Schlafs
Bedding detection device for sleep monitoring

(30) Priorité: 18.11.2013 FR 1361287
(43) Date de publication de la demande: 20.05.2015
(73) Titulaire: Withings, 92130 Issy-les-Moulineaux (FR)
(72) Inventeur: Dusanter, Florent, 92120 Montrouge (FR); Rechke, Bastien, 92310 Sevres (FR); Barrochin, Pierre, 92210 Saint Cloud (FR); Buard, Nadine, 92190 Meudon (FR); Carreel, Eric, 92190 Meudon (FR); Julien, Lodie, 92150 Suresnes (FR)
(74) Mandataire: Plasseraud IP

(56) Documents cités:
- WO-A1-2012/035737
- WO-A2-2013/033524
- JP-A- 2007 097 996
- US-A1- 2006 151 039
- US-A1- 2008 077 020
- US-A1- 2011 144 455

## Description

La présente invention est relative aux dispositifs de détection destinés à permettre la surveillance du sommeil, de tels dispositifs de détection sont habituellement installés dans la literie. Plus précisément, ce genre de dispositif de détection peut être installé au-dessus d'un matelas et sous une alèse ou une housse de matelas; il peut aussi être installé en dessous d'un surmatelas, ou en dessous du matelas principal ou en dessous de tout autre substrat; un individu à surveiller est destiné à être allongé au-dessus du dispositif de détection.

En particulier, les dispositifs les plus répandus sont formés par une chambre gonflable peu épaisse, un capteur de pression étant agencé à l'intérieur de la chambre pour mesurer les variations de pression et en déduire des informations sur le rythme cardiaque et la respiration de l'individu reposant sur le lit.

Il est connu par exemple du document US2011306844 d'avoir une chambre à air de forme générale rectangle ou carrée avec le capteur de pression agencé dans un coin de la chambre. L'accès au raccordement du capteur à l'extérieur de la literie impose à la chambre gonflable d'occuper toute la largeur du lit de manière à avoir la connexion au capteur située en bordure du lit.

Les documents US2011/0144455, JP2007/097996, WO2012/035737, WO2013/033524 divulguent également des systèmes de lits comprenant une chambre à air sous pression, sous forme de matelas ou de tube, permettant de mesurer des paramètres physiologiques d'une personne. US2006/0151039 divulgue une tige pleine élastique destinée à empêcher l'écrasement complet au niveau d'un coude d'un tuyau conduisant un fluide.

Cependant, il s'avère que la performance des chambres de telles dimensions n'est pas optimale en termes de précision et de sensibilité et de plus le coût et l'encombrement d'un tel équipement sont assez pénalisants.

Il est donc apparu un besoin de proposer une solution plus optimisée et moins encombrante pour contribuer à la surveillance du sommeil d'un individu.

A cet effet, l'invention propose un dispositif de détection, destiné à être placé sous ou sur un matelas de lit, afin de détecter par ballistographie au moins les mouvements, les rythmes cardiaque et respiratoire (par détection des des mouvements voire micro-mouvements) d'un individu reposant sur le matelas, en vue de surveiller le sommeil de l'individu, comprenant :
- une portion de captation comprenant au moins une chambre gonflable destinée à contenir un fluide formé par de l'air, destinée à être positionnée sous l'individu, en particulier sous un des points de pression de l'individu couché à savoir la tête, le buste, ou le tronc,
- une unité électronique agencée à distance de la portion de captation, comprenant au moins un transducteur de pression, destinée à être positionnée au voisinage de la bordure du matelas ou de la literie,
- une portion de transmission, interposée entre la portion de captation et l'unité électronique, comprenant au moins une conduite (autrement dit une canalisation ou encore une liaison fluide), mettant en relation fluide la chambre avec le transducteur, de sorte que l'unité électronique reçoit les changements de pression induits par les mouvements de l'individu, si petits soient-ils.

Moyennant quoi l'épaisseur du dispositif reste très limitée sous le matelas ou sur le matelas, le seul élément ayant une épaisseur potentiellement gênante est agencé en dehors de la literie (au bord du matelas voire au-delà), et pour autant le dispositif permet de capter de façon satisfaisante les mouvements de l'individu si petits soient-ils, avec une surface de captation relativement limitée, de manière optimale sur la tête ou le tronc de l'utilisateur. La détection des mouvements permet en particulier de déterminer les mouvements, le rythme cardiaque et le rythme respiratoire de l'individu.

Grâce à ces dispositions, la surface de captation est optimale et on évite d'avoir des surfaces trop importantes pour la chambre gonflable qui sont autant de volumes morts pour la fonction détection. Grâce à l'invention, les volumes morts sont moins importants, donc les variations de pression sont plus importantes et la détection de ses variations est plus facile.

Dans des modes de réalisation du dispositif selon l'invention, on peut éventuellement avoir recours en outre à l'une et/ou à l'autre des dispositions qui suivent.

La conduite de la portion de transmission peut avoir une dimension transversale très inférieure à la largeur de la chambre gonflable, de sorte que le volume de fluide dans la portion de transmission est très petit et n'influence quasiment pas la mesure.

Le dispositif de détection peut comprendre en outre une pompe pneumatique et une valve de décharge, de préférence associées à l'unité électronique, la pompe pneumatique et la valve de décharge pouvant être utilisées par l'unité électronique pour gonfler et/ou dégonfler la portion de de captation. Il est ainsi possible d'ajuster la consigne de pression grâce à la pompe et à la valve de décharge, en fonction du poids individu ainsi que du poids de la literie ou même de la localisation du dispositif de détection à l'intérieur de la literie, notamment en fonction de sa proximité avec l'individu. En outre, le système peut compenser les pertes pneumatiques éventuelles ; de plus, en associant la pompe et la valve de décharge avec l'unité électronique, un seul boîtier est à installer en bordure de literie.

Selon l'invention, il est prévu, dans la chambre gonflable, une pluralité de pontets, rendant solidaires à leur endroit les parois supérieure et inférieure de la chambre gonflable de sorte que la chambre, même lorsqu'elle est sous pression, présente une épaisseur très inférieure à sa longueur et sa largeur. Moyennant quoi on peut choisir des parois supérieure et inférieure de faible épaisseur voire de très faible épaisseur, la solidité dans le sens vertical étant procurée par les pontets ; de sorte qu'on obtient une discrétion dimensionnelle très avantageuse en termes d'épaisseur tout en autorisant le gonflage de fluide.

La distance **D** séparant la portion de captation de l'unité électronique peut être supérieure à 10 cm. Moyennant quoi la portion de captation peut être agencée de façon idéale sous le thorax, la tête ou les hanches de l'utilisateur, et l'unité électronique peut être située en dehors de la literie elle-même, préférentiellement contre le bord du lit ; de plus, la sur-longueur peut être enroulée au voisinage de l'unité électronique.

L'unité électronique peut comporter un module de mise en forme des signaux captés et un module de communication sans fil ; moyennant quoi l'unité électronique peut mettre les informations captées à disposition de toute entité distante comme une station de base, un téléphone intelligent, un serveur Web, afin de fournir à l'individu ou à un personnel de soins des informations exploitables.

Le dispositif de détection peut comprendre en outre un capteur de température et/ou un capteur d'humidité. De sorte que l'on peut complémenter les informations de mouvements par d'autres informations relatives à la qualité du sommeil de l'individu.

Le dispositif se présente à l'état dégonflé sous forme de nappe souple pliable, roulable et lessivable. On obtient ainsi un encombrement faible pour le rangement et/ou la livraison du dispositif de détection. On peut aussi lessiver à l'aide d'une éponge le dispositif de détection.

Selon l'invention, la conduite de la portion de transmission comprend une tige pleine élastique destinée à empêcher l'écrasement complet de la paroi supérieure contre la paroi inférieure. De sorte que la communication fluide reste assurée (et donc la mesure de pression fiable) quelques soient les contraintes exercées sur la portion de transmission. L'élasticité de la tige autorise par ailleurs un enroulement facile et guidé de la bande formée par la portion de transmission. Selon l'invention, un des pontets est disposé avantageusement dans l'alignement de la tige pleine au voisinage du débouché de la conduite dans la chambre gonflable ; ainsi la tige ne peut pas glisser vers l'intérieur de la chambre gonflable.

Le pontet situé dans l'alignement de la tige pleine comprend une extrémité en forme de V ouverte vers la tige pleine, apte à faire butée pour l'extrémité de la tige.

La largeur de la portion de transmission peut être sensiblement identique à la largeur de la portion de captation. Ainsi on obtient un objet facile à enrouler ; on améliore aussi le maintien à l'intérieur de la literie.

Le volume mort de gaz dans la conduite de la portion de transmission est minimal, de préférence inférieur à 5 cm³ ; de sorte que la portion de transmission a une influence quasi négligeable sur la performance de la détection des variations de pression.

L'invention vise également un kit comprenant un dispositif de détection tel que décrit précédemment et une housse démontable, le dispositif de détection étant destiné à être inséré dans la housse. Moyennant quoi la housse fait office de protection et on peut la passer à la machine à laver ; de plus la housse de protection, de par sa surface rugueuse, peut remplir la fonction de moyen de maintien dans une position correspondant à une position optimale, par exemple la tête de l'utilisateur, son thorax, ses hanches.

L'invention vise aussi un **système** de détection comprenant au moins un dispositif de détection que décrit précédemment et une entité distante. Moyennant quoi, un certain nombre d'informations relatives à la qualité du sommeil sont traitées et mises à disposition de de l'individu lui-même ou d'un personnel de soins par une entité distante.

Enfin, selon la revendication indépendante 12, l'invention vise aussi un procédé mis en oeuvre dans un dispositif tel que décrit précédemment, dans lequel l'unité électronique est configurée pour ajuster, en commandant la pompe pneumatique et la valve de décharge, la pression prévalant dans la chambre gonflable ; de sorte que l'on peut choisir la pression optimale en fonction de la configuration liée au poids de l'individu et à la position du dispositif dans la literie, et eu type de matelas.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue générale du dispositif de détection selon l'invention, intégré dans une literie,
- la figure 2 est une vue en coupe axiale, représentée en perspective du dispositif de détection de la figure 1,
- la figure 3 est une vue en coupe transversale représentée en perspective du dispositif de détection de la figure 1,
- la figure 4 illustre un schéma fonctionnel de principe,
- la figure 5 est une vue de dessus d'une variante du dispositif de détection, avec une portion de transmission plus large,
- la figure 6 montre un exemple du dispositif de détection dans sa configuration de livraison ou de rangement,
- les figures 7A et 7B représentent des configurations respectives pour un lit monoplace et pour un lit biplace,
- la figure 8 est analogue à la figure 5 et représente une variante de réalisation,
- la figure 9 montre un détail de la zone d'interface entre la portion de captation et la portion de transmission.

Sur les différentes figures, les mêmes références désignent des éléments identiques ou similaires.

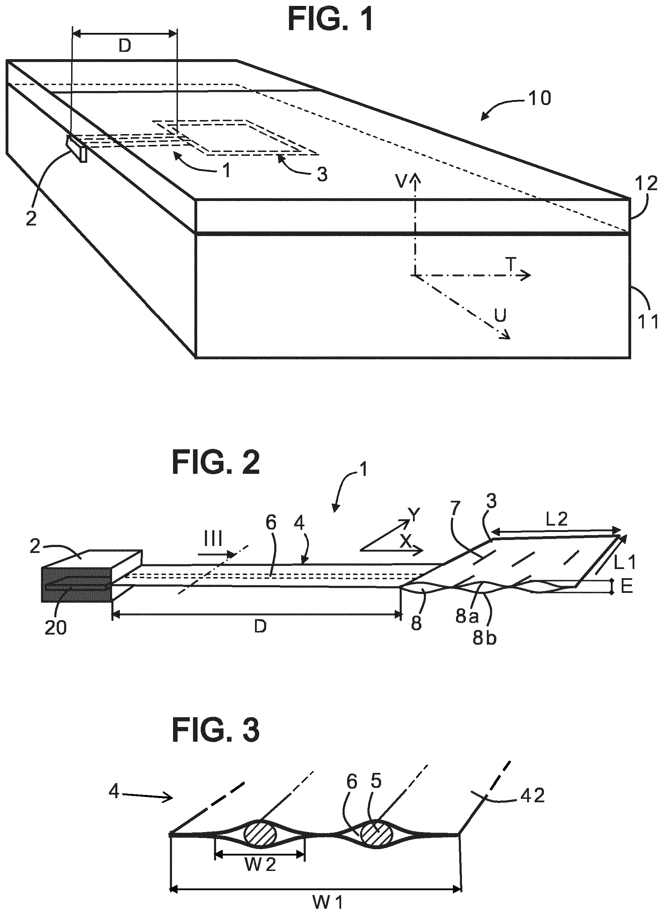

La figure 1 représente un exemple de literie 10 de lit selon la présente invention, laquelle comprend un matelas principal **11** au-dessus duquel est disposé un dispositif de détection **1** comportant une partie active de détection qui sera détaillée plus loin et dont la position correspond de façon avantageuse approximativement à un des points de pressions du corps de l'individu couché sur le matelas. Cette position optimale peut être choisie comme la zone de la tête, la zone du buste, la zone du bas du tronc (zone du bassin et des hanches). Toutefois, il s'avère que n'importe quelle position sous le tronc ou sous la tête peut quand même convenir pour une captation suffisante des mouvements de l'individu et convient pour déterminer par ballistographie, à partir changements de pression induits par les mouvements de l'individu, le rythme cardiaque et le rythme respiratoire de l'individu.

Au-dessus du dispositif de détection **1** est installé une alèse, un sur-matelas, un drap housse ou tout autre substrat **12** sur lequel peut s'installer l'individu utilisateur de la literie.

Dans une autre configuration non représentée, le dispositif de détection **1** peut aussi être installé sous le matelas principal.

Le dispositif de détection **1** est adapté pour pouvoir détecter des mouvements de l'utilisateur, y compris des mouvements de très faible amplitude, de sorte que l'analyse de ces petits mouvements permet de déterminer le niveau d'activité, le rythme cardiaque et le rythme respiratoire de l'individu, même lorsque ce dernier est immobile. L'analyse couplée des mouvements perçus, des rythmes cardiaque et respiratoire permet de distinguer les phases de sommeil et d'enregistrer les différentes phases de sommeil de l'individu.

Dans l'exemple particulier illustré aux figures 1 à 4, le dispositif de détection comprend :
- une portion de captation **3** comprenant au moins une chambre gonflable **8** destinée à contenir un fluide, notamment gazeux, particulièrement de l'air,
- une unité électronique **2,** agencée à distance de la portion de captation, comprenant au moins un transducteur de pression **26,** et une carte électronique **20,**
- une portion de transmission **4,** interposée entre la portion de captation et l'unité électronique, comprenant au moins une **conduite 6** mettant en relation fluide la chambre gonflable avec le transducteur.

De manière avantageuse, on choisit comme fluide un fluide compressible de très bonne disponibilité, en l'occurrence l'air ambiant. Moyennant quoi, on peut gonfler la portion de captation pour l'utilisation, et aussi purger la portion de captation afin de pouvoir ranger le dispositif de détection sous une forme compacte enroulée qui sera détaillée plus loin. De plus, l'utilisation d'un fluide liquide présente des risques de fuite et de mouillage de la literie.

La chambre gonflable **8** occupe la majeure partie de la portion de captation **3** ; elle a pour dimensions une largeur **L1** (selon l'axe **Y** dans les dessins), une longueur **L2** (selon l'axe **X** dans les dessins) et une épaisseur notée **E** dans la direction perpendiculaire aux axes **X** et **Y.** Lorsque le dispositif de détection est installé dans la literie, les axes X et Y se retrouvent dans le plan **T,U** du lit et la direction de l'épaisseur E de la chambre gonflable coïncide avec la direction verticale **V.**

Concernant les dimensions principales de la portion de captation, la largeur **L1** pourra être typiquement comprise entre 20 cm et 50 cm, et de façon préférentielle entre 30 cm et 40 cm. De même, la longueur **L2** pourra être typiquement comprise entre 20 cm et 50 cm, et de façon préférentielle entre 30 cm et 40 cm. Par conséquent, la portion de captation n'occupe pas forcément toute la largeur du lit ni bien entendu non plus toute la longueur du lit.

La chambre gonflable (qui dans l'exemple illustré est une chambre pneumatique) est délimitée par une paroi supérieure **8a** et une paroi inférieure **8b** qui sont soudés sur toute la périphérie de la chambre. La chambre gonflable comprend avantageusement une pluralité de pontets **7** rendant solidaires à leur endroit les parois supérieure **8a** et inférieure **8b** de la chambre gonflable. Ainsi, la chambre gonflable, même lorsqu'elle est sous pression, présente une épaisseur **E** très inférieure à sa longueur **L2** et sa largeur **L1.**

Ainsi, lorsque la chambre gonflable est dégonflée, l'épaisseur **E** sera inférieure à 8 mm, préférentiellement inférieure à 5 mm, de manière encore plus préférée inférieure à 3 mm. Il faut noter qu'une valve de purge **90** est prévue afin de pouvoir purger la chambre gonflable.

Sur un des côtés de la chambre gonflable, il est prévu une communication fluide avec la conduite **6** agencée à l'intérieur de la portion de transmission **4.**

La conduite **6** présente une dimension transversale **W2** très inférieure à largeur **L1** de la chambre, par exemple comprise entre 5 mm et 15 mm.

La largeur **W1** de la portion de transmission est aussi dans l'exemple illustré significativement inférieure à la largeur L1 de la chambre gonflable **8,** W1 est par exemple comprise entre 5 cm et 15 cm.

La distance **D,** correspondant à la longueur de la portion de transmission selon X, séparant la portion de captation **3** de l'unité électronique **2** sera typiquement supérieure à 10 cm. De plus, cette distance **D** pourra être choisie de telle manière que l'unité électronique sera suspendue contre le bord du lit, de préférence sans traîner par terre, cette distance **D** pourra être par exemple inférieure à 60 cm.

Il faut remarquer que entre la chambre gonflable et l'unité électronique, il peut y avoir une seule conduite **6** ou deux conduites séparées **6A, 6B** dont l'utilité sera vue plus loin.

Selon un aspect avantageux, visible en particulier sur la figure 3, la portion de transmission **4** comprend une tige pleine **5** élastique agencée dans la conduite **6,** destinée à empêcher l'écrasement complet de la paroi supérieure contre la paroi inférieure de la portion de transmission. Cette disposition permet d'obtenir un volume mort de fluide très faible dans la conduite, et en même temps de garantir que la communication fluide reste assurée quelques soient les contraintes exercées sur la portion de transmission. On note que la tige 5 n'est pas forcément pleine mais est réalisée en matériau résistant à l'écrasement tout en autorisant une flexion. La tige est préférentiellement de section ronde, de diamètre par exemple choisi entre 1 et 3 mm.

Grâce à la présence de cette portion de transmission **4,** on peut positionner la portion de captation **3** à distance de l'unité électronique **2,** sous l'individu, en particulier sous un des points de pression de l'individu couché à savoir la tête, le buste, ou le bas du tronc.

On prend soin de minimiser l'influence de la portion de transmission, en particulier le volume mort de gaz dans la conduite de la portion de transmission 4 sera minimal, inférieur à 10 cm³, de préférence inférieur à 5 cm³, voire inférieur à 2 cm³.

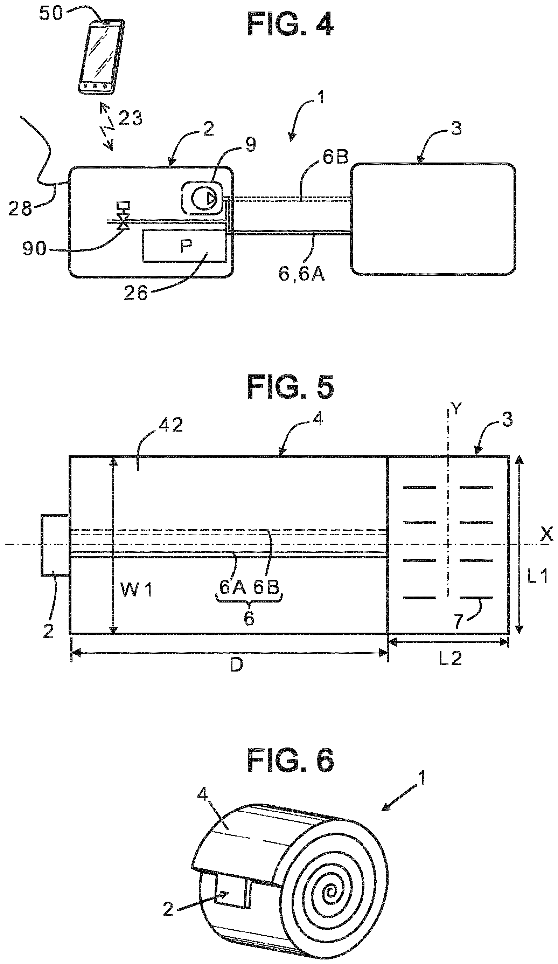

Comme illustré à la figure 4, l'unité électronique 2 comprend un transducteur de pression **26,** configuré pour convertir des signaux de pression (ou de variations de pression) en signaux électriques qui peuvent être mis en forme et être filtrés et analysés par des moyens connus en soi pour en déduire le rythme cardiaque et le rythme respiratoire de l'individu.

En outre, on peut disposer dans l'unité électronique **2** (ou à proximité immédiate) une pompe pneumatique **9** configurée pour pouvoir envoyer de l'air sous pression dans la conduite **6** (**6B** dans la configuration à deux conduites). Ainsi le système peut compenser les pertes pneumatiques éventuelles.

De plus, on peut disposer dans l'unité électronique **2** (ou à proximité immédiate) une valve de décharge **90,** sous forme d'une électrovanne. Cette électrovanne est commandée à l'ouverture pendant un laps de temps contrôlé, ce qui permet de faire baisser la pression dans la portion de captation. L'activation de la pompe et/ou de valve de décharge permet ainsi d'ajuster la consigne de pression moyenne en fonction du poids de l'individu, de la localisation du dispositif de détection à l'intérieur de la literie, notamment sa proximité avec l'individu. Plus le dispositif de détection est éloigné de l'individu dans l'épaisseur de la literie, plus la consigne moyenne de pression pourra être élevée.

De plus, lorsque la pompe est intégrée à l'unité électronique **2,** il n'y a qu'un seul boîtier à installer en bordure de literie ce qui est avantageux en terme de packaging.

L'unité électronique peut être reliée à une source d'alimentation 28, elle peut aussi comporter une interface de communication type USB. Par ailleurs, elle peut aussi comporter un moyen de communication sans fil pour échanger des données 23 avec une entité distante 50, que ce soit un téléphone intelligent, ou un ordinateur, ou une tablette, ou encore tout autre appareil adéquat.

Selon une variante de réalisation représentée à la figure 5, la portion de transmission peut présenter une largeur **W1** sensiblement identique à la largeur **L1** selon **Y** de la portion de captation, ce qui facilite l'enroulement du dispositif de détection lorsqu'on veut le ranger ou dans sa configuration de livraison, comme ceci est représenté à la figure 6. Il faut noter ici que la tige pleine **5** est suffisamment flexible pour permettre l'enrôlement sur lui-même du dispositif de détection.

Selon la présente invention, il est aussi possible d'adjoindre optionnellement au dispositif de détection un capteur de température et/ou un capteur d'humidité (non représentés) ce qui permet de compléter l'analyse de la qualité du sommeil avec les informations de température et des informations de sudation.

Il est prévu par ailleurs d'insérer le dispositif de détection, tout au moins la portion de transmission et la portion de captation dans une housse démontable (non représentée), de manière à pouvoir laver la housse indépendamment du dispositif de détection. De plus, la surface extérieure de la housse peut présenter une certaine rugosité ce qui évite un glissement de la housse par rapport à la literie.

Avantageusement, cette housse peut être munie de bande rugueuse pour fournir une accroche sur la literie et contribuer au maintien en position du dispositif de détection lorsque celui-ci est installé dans la literie.

Il faut remarquer que, dans la configuration représentée aux figures 3 et 4, la portion de transmission 4 peut comprendre deux conduites distinctes, une première conduite **6A** formant un canal de mesure de pression et une deuxième conduite **6B** formant un canal de mise en pression relié à la sortie de la pompe.

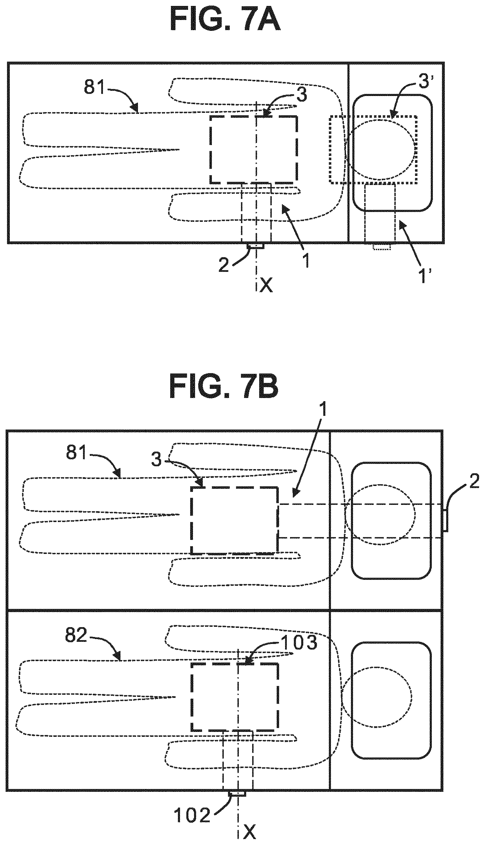

Comme illustré aux figures 7A et 7B, l'unité électronique **2** peut être positionnée contre un coté latéral du lit, ou bien sur le côté de la tête de lit. La figure 7A illustre la position **3'** de la portion de captation sous la tête l'individu pour une variante de placement du dispositif **1'**.

La figure 7B représente un lit biplace, dans lequel deux personnes prennent place. Dans cette configuration, un premier dispositif de détection **1** est placé sous le premier individu et un second dispositif de détection **100** est placé sous le second individu. Ce second dispositif de détection est identique ou similaire au premier et comprend notamment une portion de captation **103** et une unité électronique **102,** cette dernière étant configurée pour transmettre des signaux représentatifs de la qualité du sommeil du second individu vers l'entité distante **50.**

Ainsi on peut surveiller deux personnes dans un lit biplace du fait du bon ciblage de chaque dispositif de détection.

Afin de capter le plus précisément les différents mouvements de l'individu, les données électriques issues du transducteur de pression **26** sont analysées par trois voies en parallèle dans la carte électronique **20,** chaque fois présentant un filtrage spécifique, ce qui permet d'obtenir de façon la plus fiable possible le rythme cardiaque de l'individu, le rythme respiratoire de l'individu, et les mouvements effectués par l'individu pendant son sommeil.

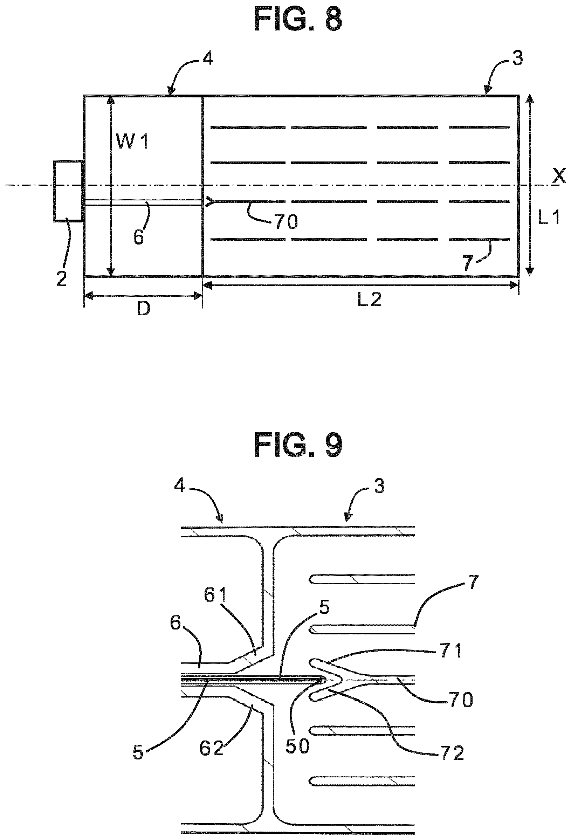

Selon une variante représentée à la figure 8, les dimensions **W1** et **L1** sont voisines ; la longueur **L2** de la portion de captation 3 est très supérieure à la longueur **D** de la portion de transmission **4.**

En outre, avantageusement, un des pontets, repéré 70 est disposé dans l'alignement la conduite **6,** et se trouve par conséquent disposé en face de l'extrémité de la tige pleine **5** au voisinage du débouché de la conduite **6** dans la chambre gonflable **8.**

Comme illustré plus en détails à la figure 9, la conduite **6** de la portion de transmission débouche dans la chambre gonflable **8** selon une embouchure évasée **61, 62.** Au centre de la conduite **6** se trouve la tige pleine 5 flexible susmentionnée qui comprend une extrémité **50** dirigée vers la chambre gonflable **8.**

Étant donné que la tige 5 est introduite dans la conduite 6 sans fixation particulière, elle risque de se déplacer longitudinalement (selon X) sous l'effet du gonflage (ou des gonflages successifs) et de (des) l'enroulement(s) de la nappe. Avantageusement, on prévoit selon l'invention qu'un des pontets empêche que la tige ne pénètre beaucoup à l'intérieur de la chambre gonflable. Selon l'exemple illustré, le pontet repéré 70 s'étend dans le prolongement longitudinal de la tige 5. De plus, son extrémité 71,72 forme un Y ou un V ouvert en direction de la tige 5, et forme ainsi une butée fiable même en cas de léger désalignement de la tige. On remarque que même si les pontets ont une direction principale orthogonale selon Y, le pontet le plus proche de l'embouchure peut faire fonction d'arrêt tout comme dans le cas illustré.

## Revendications

1. **Dispositif** de détection (1), destiné à être placé sous ou sur un matelas de lit (10), afin de détecter par ballistographie au moins les mouvements, les rythmes cardiaque et respiratoire d'un individu reposant sur le matelas, notamment pour surveiller le sommeil de l'individu, comprenant :
- une portion de captation (3) comprenant au moins une chambre gonflable (8) destinée à contenir un fluide formé par de l'air, destinée à être positionnée sous l'individu, en particulier sous un des points de pression de l'individu couché à savoir la tête, le buste, ou le bas du tronc,
- une unité électronique (2) agencée à distance de la portion de captation, comprenant au moins un transducteur de pression, destinée à être positionnée à l'extérieur de la literie elle-même, notamment au voisinage de la bordure du matelas ou de la literie,
- une portion de transmission (4), interposée entre la portion de captation et l'unité électronique, comprenant au moins une conduite (6), mettant en relation fluide la chambre gonflable avec le transducteur, de sorte que l'unité électronique reçoit les changements de pression induits par les mouvements de l'individu, **caractérisé en ce que**: la conduite de la portion de transmission (4) comprend une tige pleine (5) élastique destinée à empêcher l'écrasement complet de la paroi supérieure contre la paroi inférieure,
- la chambre gonflable (8) comprend une pluralité de pontets (7), rendant solidaires à leur endroit les parois supérieure (8a) et inférieure (8b) de la chambre gonflable, de sorte que la chambre, même lorsqu'elle est sous pression, présente une épaisseur (E) très inférieure à sa longueur (L2) et sa largeur (L1),
- un (70) des pontets (7) étant disposé dans l'alignement de la tige pleine (5) au voisinage du débouché de la conduite dans la chambre gonflable.

2. Dispositif selon la revendication 1 comprenant en outre une pompe pneumatique (9) et une valve de décharge (90), de préférence associées à l'unité électronique, la pompe pneumatique et la valve de décharge pouvant être utilisées par l'unité électronique pour gonfler et/ou dégonfler la portion de de captation (3).

3. Dispositif selon l'une des revendications 1 à 2, dans lequel la distance (D) séparant la portion de captation de l'unité électronique est supérieure à 10 cm.

4. Dispositif selon l'une des revendications 1 à 3, dans lequel l'unité électronique (2) comporte un module de mise en forme des signaux captés et un module de communication sans fil.

5. Dispositif selon l'une des revendications 1 à 4, comprenant en outre un capteur de température et/ou un capteur d'humidité.

6. Dispositif selon l'une des revendications 1 à 5, dans lequel le dispositif se présente à l'état dégonflé sous forme de nappe souple pliable, roulable et lessivable.

7. Dispositif selon l'une des revendications 1 à 6 dans lequel le pontet (70) situé dans l'alignement de la tige pleine (5) comprend une extrémité (71,72) en forme de V ouverte vers la tige pleine, apte à faire butée pour l'extrémité (50) de la tige.

8. Dispositif selon l'une des revendications 1 à 7, dans lequel la largeur (W1) de la portion de transmission est sensiblement identique à la largeur (L1) selon Y de la portion de captation.

9. Dispositif selon l'une des revendications 1 à 8, dans lequel le volume mort de gaz dans la portion de transmission (4) est minimal, de préférence inférieur à 5 cm³.

10. **Kit** comprenant un dispositif de détection selon l'une des revendications 1 à 9 et une housse, le dispositif de détection étant destiné à être inséré dans la housse.

11. **Système** de détection comprenant au moins un dispositif de détection (1) selon l'une des revendications 1 à 9, et une entité distante (50).

12. **Procédé** mis en oeuvre dans un dispositif selon la revendication 2, dans lequel l'unité électronique (2) est configurée pour ajuster, en commandant la pompe pneumatique et la valve de décharge, la pression prévalant dans la chambre gonflable.

## Patentansprüche

1. Sensorvorrichtung (1), die dazu bestimmt ist, unter oder auf einer Bettmatratze (10) angeordnet zu werden, um mittels Ballistographie zumindest die Bewegungen, den Herzrhythmus und Atmungsrhythmus eines auf der Matratze liegenden Individuums zu detektieren, insbesondere um den Schlaf des Individuums zu überwachen, aufweisend:
- einen Erfassungsteil (3) mit mindestens einer aufblasbaren Kammer (8), die dazu bestimmt ist, ein Fluid, das aus Luft besteht, zu enthalten, und dazu bestimmt ist, unterhalb des Individuums, insbesondere unterhalb eines der Druckpunkte des schlafenden Individuums, wie unterhalb des Kopfs, des Oberkörpers oder des unteren Rumpfs, angeordnet zu werden,
- eine im Abstand von dem Erfassungsteil angeordnete elektronische Einheit (2), die mindestens einen Druckwandler aufweist und dazu bestimmt ist, außerhalb des eigentlichen Betts, insbesondere in der Nähe der Matratzenkante oder der Bettkante angeordnet zu werden,
- einen zwischen dem Erfassungsteil und der elektronischen Einheit angeordneten Übertragungsteil (4), der mindestens eine Leitung (6) aufweist, die die aufblasbare Kammer in Fluidverbindung mit dem Wandler bringt, so dass die elektronische Einheit die von den Bewegungen des Individuums hervorgerufenen Druckänderungen empfängt, **dadurch gekennzeichnet, dass**
- die Leitung des Übertragungsteils (4) einen elastischen Massivstab (5) aufweist, der dazu bestimmt ist, das vollständige Niederdrücken der oberen Wand gegen die untere Wand zu verhindern,
- die aufblasbare Kammer (8) mehrere Bügel (7) aufweist, die an ihrem Ort die obere Wand (8a) und untere Wand(8b) der aufblasbaren Kammer miteinander verbinden, so dass die Kammer, selbst wenn sie unter Druck ist, eine Dicke (E) hat, die sehr klein zu ihrer Länge (L2) und ihrer Breite (L1) ist,
- in der Nähe der Mündung der Leitung in die aufblasbare Kammer einer (70) der Bügel (7) in Ausrichtung mit dem Massivstab (5) angeordnet ist.

2. Vorrichtung nach Anspruch 1, ferner aufweisend eine Pneumatikpumpe (9) und ein Ablassventil (90), die vorzugsweise an der elektronischen Einheit angeschlossen sind, wobei die Pneumatikpumpe und das Ablassventil von der elektronischen Einheit verwendet werden können, um den Erfassungsteil (3) aufzublasen und/oder daraus Luft abzulassen.

3. Vorrichtung nach einem der Ansprüche 1 bis 2, in welcher der den Erfassungsteil von der elektronischen Einheit trennende Abstand (D) größer als 10cm ist.

4. Vorrichtung nach einem der Ansprüche 1 bis 3, in welcher die elektronische Einheit (2) ein Modul zur Aufbereitung erfasster Signale und ein Modul für drahtlose Kommunikation aufweist.

5. Vorrichtung nach einem der Ansprüche 1 bis 4, ferner aufweisend einen Temperatursensor und/oder einen Feuchtigkeitssensor.

6. Vorrichtung nach einem der Ansprüche 1 bis 5, in welcher die Vorrichtung in dem luftleeren Zustand in Form einer weichen, faltbaren, zusammenrollbaren und waschbaren Decke vorliegt.

7. Vorrichtung nach einem der Ansprüche 1 bis 6, in welcher der in Ausrichtung mit dem Massivstab (5) angeordnete Bügel (70) ein Ende (71, 72) in Form eines zum Massivstab hin offenen V aufweist, das fähig ist, einen Anschlag für das Ende (50) des Stabs zu bilden.

8. Vorrichtung nach einem der Ansprüche 1 bis 7, in welcher die Breite (W1) des Übertragungsteils im Wesentlichen gleich der Breite (L1) des Erfassungsteils in Y-Richtung ist.

9. Vorrichtung nach einem der Ansprüche 1 bis 8, in welcher das Gastotvolumen in dem Übertragungsteil (4) minimal ist, vorzugsweise kleiner als 5cm³.

10. Kit, aufweisend eine Sensorvorrichtung nach einem der Ansprüche 1 bis 9 und eine Schutzhülle, wobei die Sensorvorrichtung in die Schutzhülle einzusetzen ist.

11. Sensorsystem, aufweisend mindestens eine Sensorvorrichtung (1) nach einem der Ansprüche 1 bis 9 und eine Fernentität (50).

12. Verfahren, das in einer Vorrichtung nach Anspruch 2 durchgeführt wird, in welchem die elektronische Einheit (2) konfiguriert ist, durch Steuerung der Pneumatikpumpe und des Ablassventils den in der aufblasbaren Kammer herrschenden Druck anzupassen.

## Claims

1. Detection **device** (1), intended to be placed on or under a mattress (10) so as to detect by ballistography at least the movements, heart and respiratory rates of an individual lying on the mattress, in particular in order to monitor the sleep of the individual, comprising:
- a sensing portion (3) comprising at least one inflatable chamber (8) intended to contain a fluid consisting of air, intended to be positioned under the individual, in particular under one of the pressure points of the recumbent individual namely the head, chest, or lower torso,
- an electronic unit (2) arranged at a distance from the sensing portion, comprising at least one pressure sensor, intended to be positioned externally to the bedding, in particular close to the edge of the mattress or bedding,
- a transmitting portion (4), interposed between the sensing portion and the electronic unit, comprising at least one channel (6), establishing a fluid connection between the inflatable chamber and the sensor so that the electronic unit receives the pressure changes induced by movements of the individual,
wherein the channel of the transmitting portion (4) comprises a solid flexible rod (5) preventing the complete collapse of the upper wall against the lower wall,
wherein a plurality of braces (7) are provided in the inflatable chamber, joining at the locations where they are placed the upper (8a) and lower (8b) walls of the inflatable chamber such that the chamber, even when under pressure, has a thickness (E) that is much smaller than its length (L2) and width (L1),
and wherein one (70) of the braces (7) is arranged in alignment with the solid rod (5) in the vicinity of the outlet of the channel in the inflatable chamber.

2. Device according to claim 1, further comprising an air pump (9) and a discharge valve (90), preferably associated with the electronic unit, the air pump and the discharge valve able to be used by the electronic unit to inflate and/or deflate the sensing portion (3).

3. Device according to one of claims 1 to 2, wherein the distance (D) between the sensing portion and the electronic unit is greater than 10 cm.

4. Device according to one of claims 1 to 3, wherein the electronic unit (2) comprises a module for shaping the sensed signals and a wireless communication module.

5. Device according to one of claims 1 to 4, further comprising a temperature sensor and/or a humidity sensor.

6. Device according to one of claims 1 to 5, wherein the device in its deflated state is in the form of a flexible sheet that is foldable, can be rolled up, and is washable.

7. Device according to one of claims 1 to 6, wherein the brace (70) located in alignment with solid rod (5) comprises a V-shaped open end (71,72) towards the solid rod, able to form abutment for the end (50) of the rod.

8. Device according to any one of claims 1 to 7, wherein the width (W1) of the transmitting portion is substantially the same as the width (L1) along Y of the sensing portion.

9. Device according to one of claims 1 to 8, wherein the gas dead volume within the transmitting portion (4) is minimal, preferably less than 5 cm³.

10. **Kit** comprising a detection device according to one of claims 1 to 9 and a slipcover, the detection device being intended for insertion into the slipcover.

11. Detection **system** comprising at least one detection device (1) according to one of claims 1 to 9, and a remote entity (50) .

12. **Method** implemented in a device according to claim 2, wherein the electronic unit (2) is configured to adjust, by controlling the air pump and the discharge valve, the pressure prevailing in the inflatable chamber.
